# EUROPÄISCHE PATENTSCHRIFT

(11) **EP 2 471 348 B1**
(45) Veröffentlichungstag und Bekanntmachung des Hinweises auf die Patenterteilung: **16.10.2019**
(21) Anmeldenummer: 10747603.8
(22) Anmeldetag: 27.07.2010
(51) Int. Cl.: A61N 1/44, H05H 1/24, A61B 18/00

(54) **VORRICHTUNG ZUR FLÄCHIGEN BEHANDLUNG VON BEREICHEN MENSCHLICHER ODER TIERISCHER HAUT- BZW. SCHLEIMHAUTOBERFLÄCHEN MITTELS EINES KALTEN ATMOSPHÄRENDRUCKPLASMAS**
DEVICE FOR THE PLANAR TREATMENT OF AREAS OF HUMAN OR ANIMAL SKIN OR MUCOUS MEMBRANE SURFACES BY MEANS OF A COLD ATMOSPHERIC PRESSURE PLASMA
DISPOSITIF POUR LE TRAITEMENT EN SURFACE DE ZONES SUPERFICIELLES DE LA PEAU OU DES MUQUEUSES, HUMAINES OU ANIMALES, PAR PLASMA FROID À PRESSION ATMOSPHÉRIQUE

(30) Priorität: 25.08.2009 DE 202009011521 U
(43) Veröffentlichungstag der Anmeldung: 04.07.2012
(73) Patentinhaber: Leibniz-Institut für Plasmaforschung und Technologie e.V., 17489 Greifswald (DE)
(72) Erfinder: STIEBER, Manfred, 17489 Greifswald (DE); WELTMANN, Klaus-Dieter, 18609 Binz (DE); HORN, Stefan, 17509 Loissin (DE); BRANDENBURG, Ronny, 17495 Groß Kiesow (DE); VON WOEDTKE, Thomas, 18519 Horst (DE)
(74) Vertreter: Schulz Junghans Patentanwälte PartGmbB
(86) Internationale Anmeldenummer: PCT/EP2010/060859
(87) Internationale Veröffentlichungsnummer: WO 2011/023478

(56) Entgegenhaltungen:
- EP-B1- 1 023 003
- WO-A1-2004/068916
- WO-A1-2006/001455
- WO-A2-2009/019156
- WO-A2-2009/067682
- DE-A1- 10 127 035
- DE-A1-102007 030 915
- JP-A- 2008 034 184
- US-A1- 2014 207 208
- US-B1- 7 008 596

## Beschreibung

Die Erfindung betrifft eine Vorrichtung zur flächigen Behandlung von Bereichen menschlicher oder tierischer Haut- bzw. Schleimhautoberflächen mittels eines kalten Atmosphärendruckplasmas. Kernstuck der Vorrichtung ist eine spezielle Elektrodenanordnung zur Erzeugung einer dielektrisch behinderten Oberflächenentladung, die sich flexibel auf beliebig gekrümmte Oberflächen auflegen lässt. Auf der Grundlage dieser Erfindung ist es möglich, im Bereich erkrankter Haut- bzw. Schleimhautpartien des menschlichen Körpers, in unmittelbarer Nähe der Haut- bzw. Schleimhautoberfläche oder von Wunden ein flächiges Plasma zur Behandlung der erkrankten Areale zu erzeugen, das hinsichtlich der Belastung der Haut bzw. Schleimhaut durch Temperatur und elektrische Spannungen unbedenklich ist.

### [Stand der Technik]

Im Ergebnis wissenschaftlicher Studien der letzten Jahre werden gegenwärtig für die Plasmatechnologie, die sich bereits als Schlüsseltechnologie für vielfaltige Anwendungen von Oberflächenbehandlungen in der Industrie bewährt hat, neue Applikationsmöglichkeiten in einigen Bereichen der Biologie und Medizin erschlossen ([1] M. Laroussi, "Low-Temperature Plasmas for Medicine?", IEEE Transactions on Plasma Science 2009, 37, 714-725; [2]M. Kong et al., Plasma medicine: an introductory review", New Journal of Physics 2009, 11, 115012; [3] G. Lloyd etal., "Gas Plasma: Medical Uses and Developments in Wound Care", Plasma Processes and Polymers 2010, 7, 194-211).

Das Potenzial der Plasmatechnologie für Applikationen dieser Art wird als derart bedeutend eingeschätzt, dass sich gegenwärtig international ein eigenständiges Fachgebiet, als Plasmamedizin bezeichnet, zu entwickeln beginnt. Eine wesentliche Zielstellung ist dabei beispielsweise die Entwicklung innovativer therapeutischer Methoden zur Behandlung von Haut- bzw. Schleimhauterkrankungen und chronischen Wunden mit kalten Atmosphärendruckplasmen, auf der Grundlage des Zusammenwirkens von antiseptischer Plasmawirkung und Stimulierung der Neubildung von gesundem Gewebe durch das Plasma.

Um plasmabasierte therapeutische Ansätze dieser Art systematisch erschließen und anwenden zu können, werden geeignete Plasmaquellen benötigt, die einerseits schmerzfrei arbeiten und gewährleisten, dass das zu behandelnde Gewebe nicht durch Temperatur, Austrocknung oder elektrische Spannungen geschädigt wird und andererseits flexibel für großflächige Anwendungen für verschiedene Körpereigenen unter variablen Plasmabedingungen eingesetzt werden können.

In verschiedenen Druckschriften werden Vorrichtungen zur Plasmabehandlung von lebendem Gewebe mit nichtthermischen Atmosphärenplasmen vorgeschlagen (DE 36 18 412 A1, WO 2004/105810 A1, WO 2006/116252 A2). Die in diesen Schriften beschriebenen Vorrichtungen sind mit starren Elektrodensystemen oder Düsen zur Erzeugung von Atmosphärendruckplasmen im Bereich der zu behandelnden Gewebeoberflächen ausgerüstet und gestatten daher nur die lokale Behandlung relativ kleiner Flächen. US 7008596 B1 offenbart eine Vorrichtung zur Herstellung eines Plasmas zur Erzeugung von Ozon- und / oder Sauerstoffionen in der Luft nach dem Prinzip der dielektrisch behinderten Entladung. Die Vorrichtung umfasst einen flachen, elektrisch isolierenden Träger, eine auf dem Träger angeordnete Elektrode, sowie mindestens eine elektrisch isolierende Schicht aus dielektrischem Material. Diese Vorrichtung ist allerdings nicht flexibel genug, um als Manschette zur Behandlung von Haut an die zu behandelnden Areale angeschmiegt zu werden und weist kein geeignetes Mittel für eine Gaszufuhr zur Behandlung des abgedeckten Bereichs auf. WO 2006/001455 A1 offenbart einen Mikroplasma-Generator, der aufgrund seiner nicht-flächigen Elektrode-Anordnung das gleichzeitige Beaufschlagen und zum Zwecke einer Behandlung das Abdecken von flächigen Arealen unmöglich macht. WO 2009/067682 A2 lehrt eine Vorrichtung zum Sterilisieren von Produkten mit einem flächigen Plasma. Allerdings fehlt auch dieser Vorrichtung die Eignung zur Behandlung von abgedeckten Arealen, da eine Gaszufuhr nicht vorgesehen ist. In WO 2009019156 A2 werden Vorrichtungen zur Behandlung von Oberflächen mit Plasma vorgestellt, diese Vorrichtungen sind jedoch ebenfalls nicht geeignet, ein beliebig gekrümmtes zu behandelndes Areal so abzudecken, dass es einerseits von äußeren Umwelteinflüssen abgeschottet ist und andererseits flächig mit einem Plasma beaufschlagt werden kann.

### [Aufgabe der Erfindung]

Der Erfindung liegt die Aufgabe zugrunde, eine technische Lösung zur Erzeugung eines flächigen Plasmas zu finden, die es ermöglicht, größere Flächen von Körperteilen, vor allem von beliebig gekrümmten Arealen menschlicher oder tierischer Haut bzw. Schleimhaut behandeln zu können und diese gleichzeitig vor Austrocknung zu schützen .

### [Darstellung der Erfindung]

Der Schutzbereich der Erfindung wird in Anspruch 1 festgelegt.

Die Aufgabe wird erfindungsgemäß dadurch gelöst, dass ein planares Elektrodensystem zur Erzeugung einer dielektrisch behinderten Oberflächenentladung verwendet wird, das einerseits aus flexiblen Materialien besteht, so dass es sich an gekrümmte Oberflächen anschmiegen lässt, und das andererseits eine äußere, elektrisch leitende Fläche besitzt, die als geerdete Elektrode verwendet wird und in der Weise strukturiert ist, dass sich in den frei bleibenden Zwischenräumen der Struktur dielektrisch behinderte Oberflächenentladungen ausbilden können.

Ein wesentlicher Vorteil der Erfindung, der speziell für die Wundheilung von Bedeutung ist, besteht unter anderem darin, dass durch die Ausführung als anschmiegsame Manschette eine Abdeckung des behandelten Areals erfolgt und damit ein Schutz vor Austrocknung ermöglicht wird, womit das, nach bisherigem Stand der Wissenschaft für die Wundheilung erforderliche, feuchte Milieu besser gewährleistet wird als bei der Anwendung von offenen, starren Elektrodensystemen mit evtl. erforderlichem Gasfluss. Die Ausführung als DBE (dielektrisch behinderte Entladung) mit geringem Gasfluss wirkt der Austrocknungsgefahr auch noch entgegen.

### [Beispiele]

Die Erfindung wird anhand von Figuren naher erläutert, ohne sie auf diese Figuren zu beschränken.

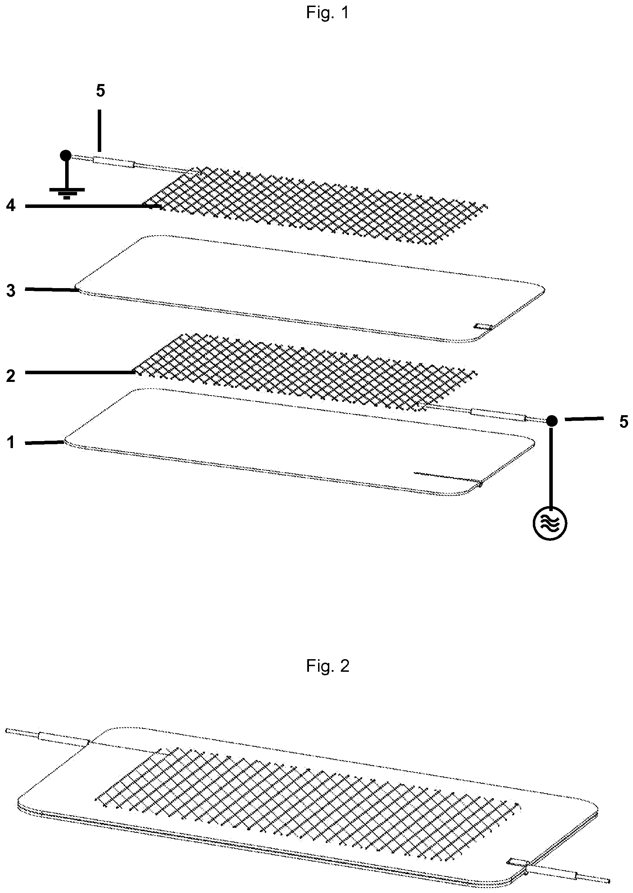

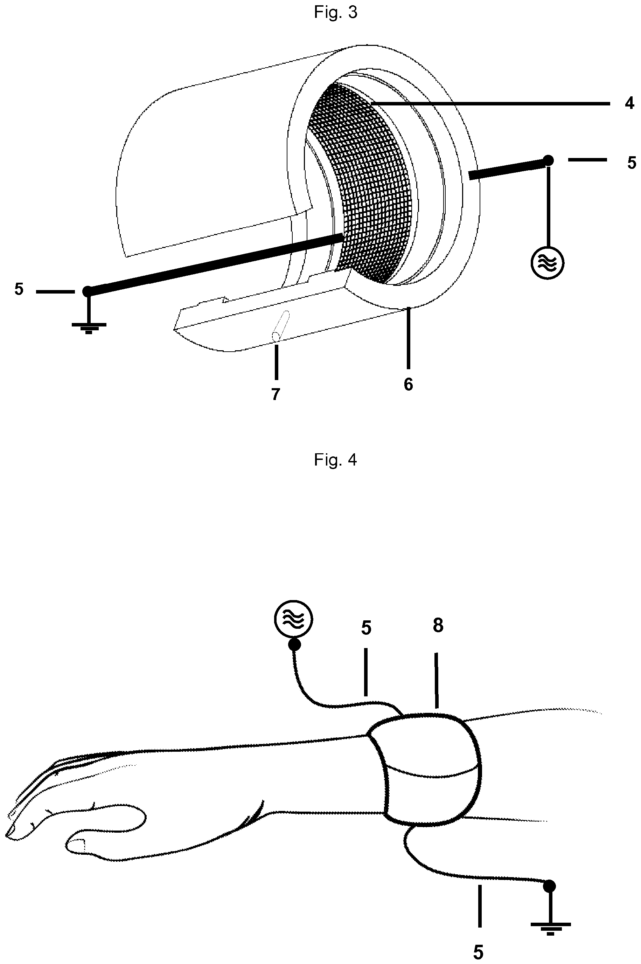

Fig. 1 und Fig. 2 zeigen hierzu den prinzipiellen Aufbau der erfindungsgemäßen Vorrichtung. Dabei ist in Fig. 2 die zusammengefügte planare Elektrodenanordnung und in Fig. 1 aus Demonstrationsgründen eine Explosionszeichnung dieser Anordnung dargestellt. Fig. 3 zeigt ein Ausführungsbeispiel in gekrümmter Form mit einem erfindungsgemäßen Gasanschluss für die optionale Zuführung eines Prozessgases und Fig. 4 als Ausführungsbeispiel eine erfindungsgemäße Armmanschette.

### [Bezugszeichenliste]

Für die nachfolgenden Zeichnungen werden folgende Bezugszeichen verwendet:
- 1: Flexibles Isoliermaterial (z.B. Elastomer, Silikonschicht)
- 2: Flexible Hochspannungs-Elektrode (z.B. Metallgaze, Metallfolie, elektrisch leitendes Elastomer)
- 3: Flexibles Dielektrikum (z.B. Elastomer, Silikonschicht)
- 4: geerdete Elektrode: flexibel und strukturiert (z.B. Metallgaze, strukturierte Metallfolie, strukturiertes, elektrisch leitendes Elastomer)
- 5: elektrisches Anschlusskabel
- 6: isolierendes Elastomer mit eingebetteter flexibler Hochspannungselektrode
- 7: Gaszufuhr
- 8: Arm-Manschette

## Patentansprüche

1. Vorrichtung zur Behandlung von Bereichen menschlicher oder tierischer Haut oder Schleimhaut mit einem kalten Atmosphärendruckplasma durch Erzeugung einer dielektrisch behinderten Oberflächenentladung, wobei die Vorrichtung als anschmiegsame Manschette zur Wundheilung ausgeführt ist, umfassend mindestens ein flexibles Isoliermaterial (1), eine planare flexible Hochspannungs-Elektrode (2), ein flexibles Dielektrikum (3), eine planare flexible geerdete Elektrode (4) und eine Gaszufuhr (7), wobei die flexible Hochspannungs-Elektrode (2) zwischen dem Isoliermaterial (1) und dem als Dielektrikum wirkenden isolierenden Elastomer (3) eingebettet ist und auf der Elastomer-Oberfläche, die der zu behandelnden Oberfläche zugewandt ist, die geerdete Elektrode (4) aufgebracht ist, **dadurch gekennzeichnet, dass** die anschmiegsame Manschette dazu ausgebildet ist, ein zu behandelndes Areal so abzudecken, dass das Areal vor Austrocknung geschützt ist.

2. Vorrichtung nach Anspruch 1, **dadurch gekennzeichnet, dass** die geerdete Elektrode (4) als Streifenstruktur oder Gitterstruktur strukturiert ist, so dass sich in den frei bleibenden Zwischenräumen der Struktur dielektrisch behinderte Oberflächenentladungen ausbilden können.

3. Vorrichtung nach Anspruch 1 oder 2, **dadurch gekennzeichnet, dass** sie zusätzlich elektrische Anschlusskabel (5) enthält.

4. Vorrichtung nach einem der Ansprüche 1 bis 3, **dadurch gekennzeichnet, dass** als flexible Hochspannungs-Elektrode (2) Metallgaze, Metallfolie oder dünne Schichten aus Metall oder leitfähiges Elastomer, vorzugsweise leitfähiges Silikon, dienen.

5. Vorrichtung nach einem der Ansprüche 1 bis 3, **dadurch gekennzeichnet, dass** als geerdete (4) Elektrode Metallgaze oder streifen- oder gitterförmige Schichten aus Metall oder leitfähigem Elastomer dienen.

6. Vorrichtung nach einem der Ansprüche 1 bis 5, **dadurch gekennzeichnet, dass** sie sich flexibel an beliebig gekrümmte Oberflächen anschmiegen lässt.

7. Vorrichtung nach einem der Ansprüche 1 bis 6, **dadurch gekennzeichnet, dass** es sich um ein Pflaster handelt.

8. Vorrichtung nach einem der Ansprüche 1 bis 6, **dadurch gekennzeichnet, dass** sie eine Arm oder Beinmanschette darstellt.

9. Pflaster nach Anspruch 7, **dadurch gekennzeichnet, dass** es Klebestellen besitzt.

## Claims

1. A device for treating areas of human or animal skin or mucous membrane by means of a cold atmospheric pressure plasma by generating a dielectric barrier surface discharge, the device being designed as a supple cuff for tissue repair, comprising at least one flexible insulating material (1), a planar flexible high-voltage electrode (2), a flexible dielectric (3), a planar flexible earthed electrode (4) and a gas supply (7), the flexible high-voltage electrode (2) being embedded between the insulating material (1) and the insulating elastomer (3) used as the dielectric, and the earthed electrode (4) being attached to the elastomer surface that faces the surface to be treated, **characterized in that** the supple cuff is designed to cover an area to be treated such that the area is protected from desiccation.

2. The device according to claim 1, **characterized in that** the earthed electrode (4) is structured as a strip structure or lattice structure, such that dielectric barrier surface discharges can form in the free intermediate spaces of the structure.

3. The device according to claim 1 or 2, **characterized in that** it additionally contains an electrical connection cable (5).

4. The device according to any one of claims 1 to 3, **characterized in that** metal gauze, metal foil or thin layers of metal or conductive elastomer, preferably conductive silicone, are used as the flexible high-voltage electrode (2).

5. The device according to any one of claims 1 to 3, **characterized in that** metal gauze or strip-shaped or lattice-shaped layers of metal or conductive elastomer are used as the earthed electrode (4).

6. The device according to any one of claims 1 to 5, **characterized in that** it can flexibly cling to any variety of curved surfaces.

7. The device according to any one of claims 1 to 6, **characterized in that** it is a bandage.

8. The device according to any one of claims 1 to 6, **characterized in that** it is an arm or leg cuff.

9. A bandage according to claim 7, **characterized in that** it comprises adhesive points.

## Revendications

1. Dispositif pour le traitement de zones de la peau ou des muqueuses, humaines ou animales, avec un plasma froid à pression atmosphérique, par génération d'une décharge de surface à barrière diélectrique, le dispositif étant conçu comme un manchon adhésif pour la cicatrisation des plaies, comportant au moins un matériau isolant souple (1), une électrode haute tension souple planaire (2), un diélectrique souple (3), une électrode de masse souple planaire (4) et une arrivée de gaz (7), dans lequel l'électrode haute tension souple planaire (2) est incorporée entre le matériau isolant (1) et l'élastomère isolant (3) agissant comme diélectrique, et l'électrode de masse (4) est appliquée sur la surface élastomère tournée vers la surface à traiter, **caractérisé en ce que** le manchon adhésif est conçu pour recouvrir une zone à traiter de manière à protéger la zone contre le dessèchement.

2. Dispositif selon la revendication 1, **caractérisé en ce que** l'électrode de masse (4) est structurée comme une structure de bande ou une structure de grille, permettant de former des décharges de surface à barrière diélectrique dans les interstices demeurant libres.

3. Dispositif selon la revendication 1 ou 2, **caractérisé en ce que** celui-ci contient en outre des câbles de raccordement électriques (5).

4. Dispositif selon l'une des revendications 1 à 3, **caractérisé en ce qu'**une gaze métallique, une feuille métallique ou de fines couches de métal ou d'élastomère conducteur, de préférence de silicone conducteur, sert/servent d'électrode haute tension souple (2).

5. Dispositif selon l'une des revendications 1 à 3, **caractérisé en ce qu'**une gaze métallique ou des couches de métal ou d'élastomère conducteur en forme de bande ou de grille sert/servent d'électrode de masse (4).

6. Dispositif selon l'une des revendications 1 à 5, **caractérisé en ce que** celui-ci adhère en souplesse à des surfaces courbées au choix.

7. Dispositif selon l'une des revendications 1 à 6, **caractérisé en ce qu'**il s'agit d'un pansement.

8. Dispositif selon l'une des revendications 1 à 6, **caractérisé en ce que** celui-ci représente un brassard ou une jambière.

9. Pansement selon la revendication 7, **caractérisé en ce que** celui-ci présente des zones de collage.
